# EUROPÄISCHE PATENTSCHRIFT

(11) **EP 1 485 135 B1**
(45) Veröffentlichungstag und Bekanntmachung des Hinweises auf die Patenterteilung: **24.08.2005**
(21) Anmeldenummer: 03712050.8
(22) Anmeldetag: 19.03.2003
(51) Int. Cl.: A61L 2/28

(54) **VERFAHREN ZUR PRÜFUNG EINER STERILISIERVERPACKUNGSEINHEIT AUF WIRKSAMKEIT GEGEN REKONTAMINATION**
METHOD FOR TESTING A STERILIZATION PACKAGING UNIT FOR ITS EFFICACY AGAINST RECONTAMINATION
PROCEDE POUR CONTROLER UNE UNITE DE CONDITIONNEMENT DE STERILISATION QUANT A SON EFFICACITE CONTRE LA CONTAMINATION

(30) Priorität: 26.03.2002 DE 10213361
(43) Veröffentlichungstag der Anmeldung: 15.12.2004
(73) Patentinhaber: DUNKELBERG, Hartmut, 37242 Bad Sooden-Allendorf (DE)
(72) Erfinder: DUNKELBERG, Hartmut, 37242 Bad Sooden-Allendorf (DE)
(74) Vertreter: Callies, Rainer Michael
(86) Internationale Anmeldenummer: PCT/EP2003/002843
(87) Internationale Veröffentlichungsnummer: WO 2003/080129

(56) Entgegenhaltungen:
- WO-A-01/13964
- DE-A- 10 006 767
- US-A- 5 922 592
- US-A- 5 955 296
- JUNGHANNSS, WINTERFELD, GABELE, KULOW: "Hygienisch-mikrobiologische und technische Überprüfung von Sterilisier-Containersystemen" ZENTRALSTERILISATION, Bd. 7, Nr. 3, 1999, Seiten 154-162, XP001153080 in der Anmeldung erwähnt

## Beschreibung

Die Erfindung bezieht sich auf ein Verfahren zur Prüfung einer zur Sterilisation von Sterilisiergut vorgesehenen Sterilisierverpackungseinheit hinsichtlich ihrer Wirksamkeit, eine mikrobielle Rekontamination des Sterilisiergutes nach dessen erfolgter Sterilisation zu verhindern. Bei dem Sterilisiergut kann es sich insbesondere um medizinisches Sterilisiergut handeln.

Insbesondere im medizinischen Bereich müssen viele Gegenstände vor ihrer Benutzung sterilisiert werden, um auf den Gegenständen vorhandene Keime, d.h. lebensfähige Mikroorganismen, abzutöten. Bei solchem medizinischem Sterilisiergut handelt es sich beispielsweise um Instrumente, Wäsche oder Flüssigkeiten. Je nach Art des Sterilisiergutes wird ein entsprechendes Sterilisationsverfahren physikalischer oder chemischer Art angewandt. Im medizinischen Bereich werden vor allem Dampfsterilisationsverfahren angewendet. Diese zeichnen sich durch eine hohe Wirksamkeit und Umweltverträglichkeit aus. Bei Dampfsterilisationsverfahren wird mit gespanntem und gesättigtem Wasserdampf sterilisiert. Die Temperatur des Wasserdampfes beträgt dabei beispielsweise 121 °C oder 134 °C. Die Sterilisation findet mittels Autoklaven statt.

Weiterhin können Keime auch durch energiereiche ionisierende Strahlen oder durch ein Plasma auf H₂O₂-Basis abgetötet werden. Zu den chemischen Sterilisationsverfahren zählen die Sterilisation mittels Ethylenoxid oder Formaldehyd.

Zur Sterilisation wird medizinisches Sterilisiergut verpackt. Dazu können Cöntainerverpackungen, beispielsweise in Form eines Koffers, Papierverpackungen, bei denen das Sterilisiergut mehrfach in Papier eingeschlagen wird, und andere Verpackungen verwendet werden. Die Verpackung dient dazu, die durch den Sterilisationsvorgang erreichte Keimfreiheit bis zum Gebrauch des Sterilisiergutes zu gewährleisten.

Die Qualitätsanforderungen an den Sterilisationsvorgang sind hoch. Entsprechend WHO-Richtlinien und verschiedenen europäischen Arzneibüchern müssen von 1 000 000 "sterilen" Produkten 999 999 keimfrei sein. Die üblichen Sterilisationsverfahren werden mit physikalisch-chemischen und mikrobiologischen Parametern evaluiert und standardisiert. Ein bekanntes und verlässliches Verfahren zur Prüfung der Wirksamkeit eines Sterilisationsvorgangs besteht darin, gleichzeitig mit dem Sterilisiergut auch eine Testkeimeinheit dem zu prüfenden Sterilisationsvorgang zu unterziehen. Bei dieser Testkeimeinheit handelt es sich um eine Anzahl von gegenüber dem Sterilisationsprozess besonders resistenten Keimen, die als Einheit durch eine Hülle verschlossen sind. Nach dem Sterilisationsvorgang wird diese Testkeimeinheit unter sterilen Bedingungen geöffnet, und es wird geprüft, ob die darin enthaltenen Keime vermehrungsfähig sind. Wenn der Sterilisationsvorgang ordnungsgemäß erfolgt ist, wird keine Vermehrung der Testkeime stattfinden, da diese Testkeime durch die Sterilisation abgetötet worden sind. Die Testkeime stellen somit einen Indikator für die Effektivität des Sterilisationsvorgangs dar.

Auch für das Verpackungsmaterial gibt es Qualitätsnormen. Diese beziehen sich in der Regel auf Teilaspekte der Verpackungseinheit wie Materialeigenschaften, Dichtigkeit von Verschlüssen, usw. Geeignete Verfahren zur Prüfung der Wirksamkeit der Verpackung als Ganzes bzw. einer in Gebrauch befindlichen Verpackungseinheit sind nicht entwickelt worden. Während wie geschildert der Sterilisationsvorgang regelmäßig auf Effektivität kontrolliert wird, gibt es jedoch für die Zeitspanne nach der Sterilisation bis zu dem Zeitpunkt des Gebrauchs des Sterilisiergutes, in der eine Rekontamination stattfinden kann, keine zufriedenstellenden Verfahren, beispielsweise mikrobiologischer Art, hinsichtlich einer Erfassung der Wirksamkeit einer Sterilisierverpackungseinheit, eine Rekontamination des Sterilisiergutes zu verhindern, bzw. hinsichtlich einer Erfassung einer möglicherweise erfolgten Rekontamination des Sterilisiergutes.

Bei einer Rekontamination des Sterilisiergutes spielt zum einen die sogenannte Keimdichtigkeit der verwendeten Verpackungseinheit eine Rolle. Diese Keimdichtigkeit ist die Fähigkeit der Verpackungseinheit, den Eintritt von Mikroorganismen zu verhindern. Zum anderen spielen für eine Rekontamination verschiedene äußere Einflüsse wie die Art der Lagerung oder des Transports und mechanische Beanspruchung des verpackten Sterilisiergutes sowie Umgebungseinflüsse wie Luftbewegung, Luftkeimgehalt und Luftdruckschwankungen eine Rolle.

Ein bekanntes Verfahren, Teilkomponenten einer Containerverpackung auf Keimdichtigkeit zu prüfen, besteht darin, über in einem Deckel des Containers vorhandene Durchlässe einen Unterdruck in dem Container zu erzeugen (EN 868-1, Anhang G). Mittels dieses Unterdrucks kann die Dichtheit einer Dichtung zwischen dem Deckel und der Containerwanne getestet werden. Hierbei handelt es sich jedoch lediglich um ein spezielles physikalisches Verfahren für Verpackungen in Gestalt von Containern, und überdies bezieht sich dieses Verfahren nur auf die Dichtung zwischen Wanne und Deckel, nicht jedoch auf die Keimdichtigkeit der Dampfdurchlässe wie Filter und Ventile. Darüber hinaus sind bei diesem Prüfverfahren Umgebungseinflüsse, wie z.B. der Luftkeimgehalt, nicht einbezogen. Somit kann durch dieses Verfahren keine sichere Aussage darüber getroffen werden, inwieweit mit einer Rekontamination von in der Verpackungseinheit enthaltenem Sterilisiergut gerechnet werden muss.

Ferner gibt es ein mikrobiologisches DIN-Prüfverfahren (DIN 58953, Teil 6), das zur Prüfung von Sterilisationspapier vorgesehen ist. Bei diesem Verfahren wird ein Keimdurchlässigkeitsprüfgerät in Form einer Laborglasflasche mit dem zu prüfenden Sterilisationspapier verschlossen und geprüft, ob durch Abkühlung der Luft im Keimdurchlässigkeitsgerät ein Luftstrom in die Laborglasflasche gelangt, der keimhaltige Partikel durch das Sterilisationspapier transportiert, wenn dieses vor dem Abkühlen mit Sporenerde beschickt wurde. Dabei ist vorgesehen, die Laborglasflasche mehrfach hintereinander auf 50 °C zu erwärmen und auf 10 °C abzukühlen. Eventuell durch das Sterilisationspapier in die Laborglasflasche eintretende Sporen werden mikrobiologisch mittels Bebrütung der Nährmedium enthaltenden Laborglasflasche erfasst und ausgewertet. Dieses Prüfverfahren bezieht sich jedoch nicht auf eine Verpackungseinheit als Ganzes, sondern nur auf Papier, das als Material für Verpackungen verwendet wird. Es handelt sich um eine reine Materialprüfung, bei der abgesehen von dem Aufbringen von Sporenerde keine weiteren Umgebungseinflüsse einbezogen werden.

Weiter ist bekannt, stichprobenhaft Teile des Sterilisiergutes nach einer bestimmten Zeitspanne auf Rekontamination zu testen. Nachteilig hierbei ist jedoch die Tatsache, dass dazu die Verpackungseinheit geöffnet werden muss und bei der Untersuchung selbst Keime auf den Untersuchungsgegenstand gelangen können, so dass das Untersuchungsergebnis dadurch fehlerbehaftet sein kann. Für den Krankenhausbetrieb ist dieses Verfahren jedoch nicht praktikabel, so dass es routinemäßig in Krankenhäusern keine Anwendung findet.

Aus der Publikation von de Bruijn, A.C.P. und Kastelein, J., Einfach- oder Mehrfach-Verpackung von Medizinprodukten: Verfahrensbewertung durch Forschung, Zentraisterilisation 1999; 7 (5): 292-303 ist ein Verfahren bekannt, mit dem die Filterwirkung einer Verpackung gegenüber einem Aerosol aus 1,0 µm Latexpartikeln mittels eines Partikelzähigerätes (vgl. a.a.O., 3., S. 297, 1. Abs.) bestimmt wird. Das bekannte Verfahren ist nicht dazu bestimmt und nicht geeignet, Verpackungen in ihrer Gebrauchsform auf ihr Vermögen zu untersuchen, Sterilität unter Bedingungen der Krankenhauspraxis zu erhalten. Insbesondere beruht das Verfahren nicht auf der Bestimmung des relevanten Endpunktes, nämlich der Erfassung der mikrobiologischen Kontamination.

Aus der Publikation von Junghannß U., Winterfeld S., Gabele L., Kulow U., Hygienisch-mikrobiologische und technische Überprüfungen von Sterilisier-Containersystemen, Zentralsterilisation 1999; 7 (3): 154-162 ist ein Verfahren zur Prüfung von Sterilisierbehättern in Form von Verpackungscontainern bekannt. Bei diesem Verfahren wird eine Testkammer verwendet, in welche die zu prüfenden Container eingebracht werden können. Vor Einbringung in die Testkammer wurden die Container mit den dazugehörigen Filtern beschickt und sterilisiert. Anschließend wurden die Container mit Nährboden in Petrischalen versehen und wieder verschlossen. Mittels einer Sprühflasche wurde in das Innere der Testkammer ein keimhaltiges Aerosol gegeben. Über einen Anschlussstutzen, mit dem die zu prüfenden Container vor Einbringung in die Testkammer versehen worden waren, erfolgte eine Absaugung mittels einer Schlauchpumpe durch die zu überprüfenden Container hindurch. Nach dem Absaugen wurden die Container wiederum geöffnet, und die Nährbodenschalen wurden entnommen. Anschließend wurden diese zum Nachweis von Keimen auf dem Nährboden bebrütet. Daran anschließend erfolgte das Auszählen von koloniebildenden Einheiten (KBE).

Das bekannte Verfahren eignet sich nicht dazu, eine Endpackung in der Form, wie sie in der Praxis eingesetzt wird, auf ihre Barrierewirksamkeit bezüglich einer Rekontamination zu prüfen. Vielmehr müssen die Container vor Einbringung in die bekannte Testkammer mit einem Ansaugstutzen versehen werden. Ferner müssen die Container, nachdem sie in der Testkammer in Kontakt mit dem Aerosol waren, aus der Testkammer herausgenommen und geöffnet werden, um die Nährbodenschalen zu entnehmen und in einer separaten Vorrichtung zu bebrüten. Dadurch ist verhindert, dass der Container im ungestörten Zustand bis zur Auswertung der Untersuchungsergebnisse bleibt. Somit sind äußere Fehlerquellen bei der Untersuchung nicht auszuschließen. Die vorgesehene Entnahme der Nährbodenschalen für die nachfolgende Bebrütung ist vielmehr mit einem Kontaminationsrisiko und somit mit dem Risiko einer Messungenauigkeit verbunden.

Nach Junghannß et al. wird lediglich ein Differenzwert für die Keimundurchlässigkeit bestimmt, jedoch kein Absolutwert ermittelt. Bei der bekannten Versuchsanordnung konnte keine Aussage zur Keimfreiheit (Sterilität) aufgrund des unsterilen Umgangs mit dem Nährboden gemacht werden.

Der Erfindung liegt daher die Aufgabe zugrunde, ein einfach zu handhabendes und effektives gattungsgemäßes Verfahren zur Prüfung einer Sterilisierverpackungseinheit als Ganzes hinsichtlich ihrer Wirksamkeit, eine Rekontamination des Sterilisiergutes nach dessen erfolgter Sterilisation zu verhindern, zur Verfügung zu stellen.

Bei dem erfindungsgemäßen Verfahren wird die Verpackungseinheit mit Nährmedium beladen, einem Sterilisationsvorgang unterzogen und unter Umgebungsbedingungen über einen Zeitraum gelagert. Anschließend wird die Verpackungseinheit Bedingungen, die eine Vermehrung von Keimen bzw. Mikroorganismen in oder auf dem Nährmedium fördern, zur Kultivierung von möglicherweise in oder auf dem Nährmedium vorhandenen, lebensfähigen Keimen ausgesetzt. Hierbei kann es sich insbesondere um eine Bebrütung, beispielsweise in einem Brutschrank oder einem Brutraum, handeln. Zu den Vermehrungsbedingungen gehören z.B. eine für die Vermehrung zu erwartender Keime günstige Temperatur sowie eine günstige Gasatmosphäre. Diese Kultivierung geschieht in der Regel über eine vorgegebene Zeit. Anschließend wird die Verpackungseinheit geöffnet, und Keime, die sich in oder auf dem Nährmedium vermehrt haben, werden ermittelt. Sofern nach dem Sterilisationsvorgang, von dessen ordnungsgemäßem Ablauf ausgegangen wird bzw. dessen ordnungsgemäßer Ablauf mittels der oben beschriebenen Testkeimindikatoren geprüft worden ist, eine Kontamination des Nährmediums durch Eintritt von Keimen in die Verpackungseinheit und Ansiedlung in oder auf dem Nährmedium stattgefunden hat, sind die Keime wie oben beschrieben kultiviert worden. Wenn in oder auf dem Nährmedium sich vermehrende Keime gefunden werden, ist dies ein klarer Beleg dafür, dass in die Sterilisierverpackungseinheit nach dem Sterilisationsvorgang Keime eingedrungen sind. Dies kann darauf zurückzuführen sein, dass die Sterilisierverpackungseinheit grundsätzliche Mängel aufweist, oder es kann auch darauf zurückzuführen sein, dass die Umgebungsbedingungen, wie z.B. Luftkeimgehatt und Nässe, in Bezug auf eine Vermeidung von Rekontamination sehr ungünstig waren.

Das erfindungsgemäße Verfahren ist einfach zu handhaben und erlaubt eine umfassende Prüfung einer Sterilisierverpackungseinheit bzw. einer grundsätzlichen Konstruktion einer Sterilisierverpackungseinheit hinsichtlich ihrer Wirksamkeit, eine Rekontamination von Sterilisiergut zu verhindern. Diese Prüfung ist deshalb umfassend, weil anders als beim Stand der Technik nicht nur Teilfunktionen wie die Dichtheit zwischen Deckel und Wanne einer speziellen Containerverpackungseinheit oder die Keimdurchlässigkeit von Sterilisationspapier geprüft werden, sondern die Keimdichtigkeit der gesamten Verpackungseinheit in Abhängigkeit von Material-, Form- und Konstruktionsmerkmalen, und weil auch die speziellen Umgebungsbedingungen, die das Rekontaminationsrisiko beeinflussen, in die Prüfung eingehen. Insbesondere ist es bei diesem Prüfverfahren unmöglich, durch ein Öffnen der Verpackung fehlerbehaftete Prüfergebnisse zu erhalten.

Zur Bestimmung des Einflusses der Umgebungsbedingungen auf das Risiko einer Rekontamination ist es vorteilhaft, die Umgebungsbedingungen und den Zeitraum der Lagerung der Verpackungseinheit zwischen dem Sterilisationsvorgang und dem Kultivierungsschritt möglichst definiert vorzugeben.

Die Verpackungseinheit kann zusätzlich zu dem Nährmedium mit Sterilisiergut bestückt werden. Wenn in oder auf dem Nährmedium nach dem Kultivierungsschritt vermehrungsfähige Keime gefunden werden, muss davon ausgegangen werden, dass auch das Sterilisiergut in einem korrelierten Maße rekontaminiert ist. Wenn hingegen das Nährmedium, in oder auf dem die Keime in Verbindung mit den während der Kultivierung gegebenen Umgebungsbedingungen sehr gute bis optimale Lebensbedingungen haben, keine sich vermehrenden Keime aufweist, kann zuverlässig davon ausgegangen werden, dass das Sterilisiergut nicht rekontaminiert ist. Das kultivierte Nährmedium besitzt somit eine Indikatorfunktion hinsichtlich der Rekontamination von Sterilisiergut. Diese Ausführungsform des erfindungsgemäßen Verfahrens ist somit einer stichprobenhaften und wenig aussagefähigen Prüfung von Teilen des Sterilisiergutes eindeutig überlegen. Insbesondere ist das Prüfergebnis gemäß dem erfindungsgemäßen Verfahren sehr viel weniger fehlerbehaftet als die bekannte stichprobenhafte Prüfung, bei der durch den Prüfvorgang selbst eine Kontamination des Prüfgegenstandes stattfinden kann.

Der Sterilisationsvorgang kann insbesondere in Form einer Dampfsterilisation erfolgen. Aber auch andere Sterilisationsverfahren sind denkbar, wie z.B. Sterilisation mittels energiereicher ionisierender Strahlen oder bestimmter chemischer Wirkstoffe wie Ethylenoxid oder Formaldehyd.

Bei dem Nährmedium kann es sich um ein Standardmedium oder um ein Selektivnährmedium, das insbesondere für das Wachstum bzw. die Vermehrung bestimmter Keime ausgelegt ist, handeln. Vorzugsweise ist das Nährmedium ein Nährboden, so dass die Verpackungseinheit mit dem darin befindlichen Nährboden ohne Probleme transportiert werden kann. Üblich und günstig ist ein Nährboden auf Agarbasis. Im Falle einer Dampfsterilisation und der Verwendung eines Nährbodens auf Agarbasis sollte ein Sterilisationsprogramm gewählt werden, das gewährleistet, dass es im Anschluss an die Sterilisierphase durch Anlegen eines Vakuums nicht durch Siedeverzug zum Überkochen des noch flüssigen Agars kommen kann.

Bei Verwendung eines Nährbodens kann als Maß der Wirksamkeit der Verpackungseinheit gegen eine Rekontamination die Anzahl von auf dem Nährboden gewachsenen Kolonien durch Auszählung bestimmt werden. Somit kann also auch eine quantitative Aussage hinsichtlich der Barrierewirkung der Verpackungseinheit unter vorgegebenen Umgebungsbedingungen getroffen werden.

Der Nährboden kann in eine thermostabile Schale aus Glas oder Kunststoff eingebracht sein. Mit solchen Schalen kann die gesamte Grundfläche der Verpackungseinheit abgedeckt sein. Grundsätzlich ist es auch denkbar, den Nährboden direkt in eine Containerverpackung zu geben, wenn diese einen geschlossenen Boden aufweist, also insbesondere nicht mit einem Ventil zum Ablauf von Kondenswasser versehen ist. Durch die Einbringung von Nährboden in Schalen in die Verpackungseinheit ist die Handhabung des Nährbodens besonders einfach. Die genannte Abdeckung der Grundfläche der Verpackungseinheit mit Nährboden ermöglicht eine besonders hohe Zuverlässigkeit des Verfahrens.

Wenn bei dem Sterilisationsvorgang ein Sterilisationsverfahren angewendet wird, das mit einer Temperaturerhöhung des Inhalts der Verpackungseinheit verbunden ist, sollte man die Verpackungseinheit abkühlen lassen, bevor sie unter den Umgebungsbedingungen über den Zeitraum gelagert wird. Bei Verwendung eines Nährbodens auf Agarbasis sollte die Verpackungseinheit auf unter 40 °C abkühlen, da Agar bei Temperaturen von mehr als 40 °C verflüssigt.

Bei der Verpackungseinheit kann es sich um eine Containerverpackung, eine Papierverpackung oder eine andere Verpackung handeln. Bei der Papierverpackung kann beispielsweise ein Sterilisiersieb verwendet werden, auf dem zur Sterilisation vorgesehene Instrumente angeordnet sind.

Ferner kann erfindungsgemäß vorgesehen sein, eine zur Sterilisation von Sterilisiergut, insbesondere medizinischem Sterilisiergut, vorgesehene Sterilisierverpackungseinheit hinsichtlich ihrer Wirksamkeit, eine mikrobielle Rekontamination des Sterilisiergutes nach dessen erfolgter Sterilisation zu verhindern, in Abhängigkeit von variablen Umgebungsbedingungen zu prüfen, indem mehrfach hintereinander eines der oben beschriebenen Verfahren angewendet wird, wobei jedoch die Umgebungsbedingungen, unter denen die Verpackungseinheit über den Zeitraum gelagert wird, bei den einzelnen Anwendungen des Verfahrens unterschiedlich sind. Es können auch mehrere gleiche Muster eines Typs von Verpackungseinheit gleichzeitig den obigen, sich nur in bezug auf die Umgebungsbedingungen unterscheidenden Verfahren unterzogen werden.

Dieses Verfahren erlaubt, einen möglichst hohen Schutz gegen Rekontamination von Sterilisiergut, das sich in einem bestimmten Typ von Sterilisierverpackungseinheit befindet, zu erreichen, indem Umgebungsbedingungen gefunden werden, die in Verbindung mit der Sterilisierverpackungseinheit den gewünschten Schutz gegen Rekontamination gewährleisten. Auf diese Weise kann eine Aussage über die erforderliche hygienische Qualität eines Raumes, in dem die Verpackungseinheit gelagert wird, gemacht werden bzw. ein Anforderungsprofil an einen solchen Raum bestimmt werden. Es gelingt also mit dem Verfahren, raumluft-hygienische Kenngrößen für Parameter wie Luftkeimgehalt, aber auch Kenngrößen für Parameter von Transportbedingungen wie Erschütterungen festzulegen. Somit sind bei dieser Ausführungsform der Erfindung sowohl die Sterilisierverpackungseinheit als auch die Umgebungsbedingungen Parameter, die es gilt, entsprechend dem übergeordneten Ziel eines möglichst hohen Schutzes gegen Rekontamination oder eines bestimmten Schutzgrades unter Optimierung des Aufwandes einzustellen. Die Art der Verpackungseinheit und die Umgebungsbedingungen werden dabei in Beziehung zueinander gesetzt bzw. untersucht.

Eine Prüfung dieser umfassenden Art, bei der Anforderungen an eine Raumqualität in Abhängigkeit von einer bestimmten Verpackungseinheit ermittelt werden können, wird erst durch die vorliegende Erfindung ermöglicht.

Im Folgenden wird die Erfindung anhand eines Ausführungsbeispiels näher erläutert.

### Ausführungsbeispiel

Ein wiederverwendbarer Sterilisierbehälter für Dampf-Sterilisatoren nach EN 285 soll in Bezug auf seine Wirksamkeit zur Verhinderung einer Rekontamination von Sterilisiergut untersucht werden. Bei dem Behälter handelt es sich um eine Containerverpackung der Größe 596 x 275 x 115 mm mit einem Deckel, der einen Filter aufweist. Zur Vorbereitung der Untersuchung des Containers werden zwei offene, thermostabile Schalen (Größe 175 x 225 mm) mit je 300 ml Nährmedium CASO-Agar (Fa. Merck) in eine Siebschale (515 x 245 x 60 mm) gelegt. Die beladene Siebschale wird in die Wanne des Containers eingebracht, und der Container wird mit dem Deckel verschlossen.

Anschließend wird der Container einem Sterilisationsvorgang unterzogen. Dazu wird ein Dampfsterilisator (Autoklav) verwendet. Die Sterilisiertemperatur beträgt 121 °C, die Sterilisierzeit 15 min. Als Sterilisationsprogramm wird ein Programm für zu sterilisierende Lösungen verwendet. Ein solches Programm hat den Vorteil, dass es nicht zum Überkochen des noch flüssigen Agars durch Siedeverzug kommt.

Nach dem Sterilisationsvorgang wird der Container entweder unter praxisüblichen Bedingungen transportiert und gelagert oder der Container wird bezüglich Luftkeimgehalt, Luftdruckschwankungen und Transportweg definierten experimentellen Bedingungen ausgesetzt. Die Expositionsdauer wird mit einem Wert im Bereich von ca. 1-4 Tagen gewählt.

Anschließend wird der weiterhin geschlossene Container zur Kultivierung bei 37 °C für 48 h in einen Brutschrank eingebracht.

Nach dieser Kultivierung werden auf dem Nährboden gewachsene Kolonien ausgezählt. Bei diesen Kolonien handelt es sich um Kolonie-bildende Einheiten (KBE), deren Anzahl der Zahl der durch Rekontamination eingedrungenen Keime entspricht. Daher ist die Zahl der Kolonien ein Maß für die Rekontamination und damit die Wirksamkeit des Containers als Barriere zur Sicherung der Keimfreiheit.

## Patentansprüche

1. Verfahren zur Prüfung einer zur Sterilisation von Sterilisiergut, insbesondere medizinischem Sterilisiergut, vorgesehenen Sterilisierverpackungseinheit hinsichtlich ihrer Wirksamkeit, eine mikrobielle Rekontamination des Sterilisiergutes nach dessen erfolgter Sterilisation zu verhindern, **dadurch gekennzeichnet, dass**
- die Verpackungseinheit mit Nährmedium beladen wird,
- die Verpackungseinheit einem Sterilisationsvorgang unterzogen wird,
- die Verpackungseinheit unter Umgebungsbedingungen über einen Zeitraum gelagert wird,
- die Verpackungseinheit Vermehrungsbedingungen von Keimen zu einer Kultivierung von in oder auf dem Nährmedium vorhandenen Keimen ausgesetzt wird,
- die Verpackungseinheit geöffnet wird und Keime, die sich in oder auf dem Nährmedium vermehrt haben, ermittelt werden.

2. Verfahren nach Anspruch 1,
**dadurch gekennzeichnet, dass** bei der Lagerung der Verpackungseinheit die Umgebungsbedingungen und der Zeitraum vorgegeben werden.

3. Verfahren nach Anspruch 1 oder 2,
**dadurch gekennzeichnet, dass** die Verpackungseinheit vor dem Sterilisationsvorgang zusätzlich zu dem Nährmedium mit Sterilisiergut beladen wird.

4. Verfahren nach einem der vorhergehenden Ansprüche,
**dadurch gekennzeichnet, dass** bei dem Sterilisationsvorgang Dampfsterilisation angewendet wird.

5. Verfahren nach einem der vorhergehenden Ansprüche,
**dadurch gekennzeichnet, dass** es sich bei dem Nährmedium um ein Selektivnährmedium handelt.

6. Verfahren nach einem der vorhergehenden Ansprüche,
**dadurch gekennzeichnet, dass** es sich bei dem Nährmedium um einen Nährboden, insbesondere auf Agarbasis, handelt.

7. Verfahren nach Anspruch 6,
**dadurch gekennzeichnet, dass** als Maß der Wirksamkeit der Verpackungseinheit gegen Rekontamination die Anzahl von auf dem Nährboden gewachsenen Kolonien, von denen jede auf einen sie auslösenden Keim zurückgeführt wird, bestimmt wird.

8. Verfahren nach Anspruch 6 oder 7,
**dadurch gekennzeichnet, dass** der Nährboden sich in einer thermostabilen Schale aus Glas oder Kunststoff befindet.

9. Verfahren nach Anspruch 8,
**dadurch gekennzeichnet, dass** eine Grundfläche der Verpackungseinheit durch den Nährboden enthaltende Schalen abgedeckt wird.

10. Verfahren nach einem der vorhergehenden Ansprüche 6 bis 9,
**dadurch gekennzeichnet, dass** man bei Verwendung eines Nährbodens auf Agarbasis die Verpackungseinheit nach dem Sterilisationsvorgang, wenn dieser mit einer Temperaturerhöhung verbunden ist, auf unter 40 °C abkühlen lässt, bevor die Verpackungseinheit unter den Umgebungsbedingungen über den Zeitraum gelagert wird.

11. Verfahren zur Prüfung einer zur Sterilisation von Sterilisiergut, insbesondere medizinischem Sterilisiergut, vorgesehenen Sterilisierverpackungseinheit hinsichtlich ihrer Wirksamkeit in Abhängigkeit variabler Umgebungsbedingungen, eine mikrobielle Rekontamination des Sterilisiergutes nach dessen erfolgter Sterilisation zu verhindern,
**dadurch gekennzeichnet, dass** ein Verfahren nach einem der vorhergehenden Ansprüche mehrfach hintereinander mit unterschiedlichen Umgebungsbedingungen, unter denen die Verpackungseinheit über den Zeitraum gelagert wird, durchgeführt wird.

## Claims

1. Method for testing a sterilization packaging unit provided for sterilization of objects to be sterilized, in particular medical objects to be sterilized, for its efficacy against microbial recontamination of the sterilized objects after they have been sterilized, **characterized in that**
- the packaging unit is charged with nutrient medium,
- the packaging unit is subjected to a sterilization process,
- the packaging unit is stored for a period of time under ambient conditions,
- the packaging unit is subjected to conditions in which microbes multiply for cultivation of microbes which are present in or on the nutrient medium,
- the packaging unit is opened and microbes, which have multiplied in or on the nutrient medium, are observed.

2. Method as claimed in claim 1, **characterized in that** when storing the packaging unit the ambient conditions and the period of time are preset.

3. Method as claimed in claim 1 or 2, **characterized in that** prior to the sterilization process the packaging unit is charged with objects for sterilization in addition to the nutrient medium.

4. Method as claimed in any one of the preceding claims, **characterized in that** vapor sterilization is used in the sterilization process.

5. Method as claimed in any one of the preceding claims, **characterized in that** the nutrient medium is a selective nutrient medium.

6. Method as claimed in any one of the preceding claims, **characterized in that** the nutrient medium is a solid nutrient medium, in particular an agar-based solid nutrient medium.

7. Method as claimed in claim 6, **characterized in that** the number of colonies grown on the solid nutrient medium, each of which is attributed to a microbe initiating the colony, is determined as a measure of the efficacy of the packaging unit against recontamination.

8. Method as claimed in claim 6 or 7, **characterized in that** the solid nutrient medium is located in a thermostable dish made from glass or synthetic material.

9. Method as claimed in claim 8, **characterized in that** a bottom surface of the packaging unit is covered by dishes containing the solid nutrient medium.

10. Method as claimed in any one of the preceding claims 6 to 9, **characterized in that** when using an agar-based solid nutrient medium the packaging unit is allowed to cool to below 40°C after the sterilization process, when this sterilization process is associated with an increase in temperature, before the packaging unit is stored for the period of time under the ambient conditions.

11. Method for testing a sterilization packaging unit provided for sterilization of objects to be sterilized, in particular medical objects to be sterilized, for its efficacy, in dependence upon variable ambient conditions, against microbial recontamination of the sterilized objects after they have been sterilized,
**characterized in that** a method in accordance with any one of the preceding claims is carried out repeatedly and consecutively with different ambient conditions under which the packaging unit is stored over the period of time.

## Revendications

1. Procédé destiné à contrôler sur une unité de conditionnement de stérilisation, prévue pour la stérilisation d'un produit à stériliser, en particulier des produits médicaux à stériliser, l'efficacité de ladite unité contre une recontamination microbienne dudit produit après que celui-ci a été correctement stérilisé, **caractérisé en ce que**
- un milieu nutritif est introduit dans l'unité de conditionnement,
- l'unité de conditionnement est soumise à un processus de stérilisation,
- l'unité de conditionnement est stockée dans des conditions d'environnement pendant une période donnée,
- l'unité de conditionnement est soumise à des conditions de multiplication des germes en vue d'une culture des germes présents dans ou sur le milieu nutritif,
- on ouvre l'unité de conditionnement et on détermine les germes qui se sont multipliés dans ou sur le milieu nutritif.

2. Procédé selon la revendication 1, **caractérisé en ce que**, pour le stockage de l'unité de conditionnement, les conditions d'environnement et la période de stockage sont prédéfinis.

3. Procédé selon la revendication 1 ou 2, **caractérisé en ce que**, en plus du milieu nutritif, un produit à stériliser est introduit dans l'unité de conditionnement avant le processus de stérilisation.

4. Procédé selon l'une quelconque des revendications précédentes, **caractérisé en ce que** la stérilisation à la vapeur est appliquée pour le processus de stérilisation.

5. Procédé selon l'une quelconque des revendications précédentes, **caractérisé en ce que** le milieu nutritif est un milieu nutritif sélectif.

6. Procédé selon l'une quelconque des revendications précédentes, **caractérisé en ce que** le milieu nutritif est un bouillon de culture, en particulier à base d'agar-agar.

7. Procédé selon la revendication 6, **caractérisé en ce que** le nombre de colonies développées sur le bouillon de culture, parmi lesquelles chacune résulte d'un germe les engendrant, est déterminé en tant que critère de l'efficacité de l'unité de conditionnement contre la recontamination.

8. Procédé selon la revendication 6 ou 7, **caractérisé en ce que** le bouillon de culture est disposé dans une coupe thermostable en verre ou en matière plastique.

9. Procédé selon la revendication 8, **caractérisé en ce qu'**une surface de base de l'unité de conditionnement est recouverte par des coupes contenant le bouillon de culture.

10. Procédé selon l'une quelconque des revendications précédentes 6 à 9, **caractérisé en ce que**, en cas d'utilisation d'un bouillon de culture à base d'agar-agar, on laisse refroidir l'unité de conditionnement à une température inférieure à 40°C après le processus de stérilisation, lorsque celui-ci est lié à une augmentation de température, avant de stocker l'unité de conditionnement dans des conditions d'environnement et pendant la période de stockage.

11. Procédé destiné à contrôler sur une unité de conditionnement de stérilisation, prévue pour la stérilisation d'un produit à stériliser, en particulier des produits médicaux à stériliser, l'efficacité de ladite unité en fonction de conditions d'environnement variables, contre une recontamination microbienne dudit produit après que celui-ci a été correctement stérilisé, **caractérisé en ce qu'**un procédé selon l'une quelconque des revendications précédentes est mis en oeuvre plusieurs fois successivement dans des conditions d'environnement différentes, dans lesquelles l'unité de conditionnement est stockée pendant la période de stockage.
